# EUROPEAN PATENT APPLICATION

(11) **EP 4 079 865 A1**
(43) Date of publication of application: **26.10.2022**
(21) Application number: 21382341.2
(22) Date of filing: 20.04.2021
(51) Int. Cl.: C12Q 1/6809

(54) **METHOD FOR ASSESSING THE INTEGRITY OF A CIRCULAR DNA MOLECULE**

(71) Applicant: Servizo Galego de Saude, 15703 Santiago de Compostela (ES); Fundación Profesor Novoa Santos, 15006 A Coruña (ES)
(72) Inventor: MOSQUERA REY, Alejandro, 15006 A Coruña (ES); BLANCO GARCÍA, Francisco Javier, 15006 A Coruña (ES); REGO PÉREZ, Ignacio, 15006 A Coruña (ES); FERNÁNDEZ GARCÍA, José Luis, 15006 A Coruña (ES); OTERO FARIÑA, Fátima, 15006 A Coruña (ES); GUILLÉN FAJARDO, Rebeca María, 15006 A Coruña (ES)
(74) Representative: Hoffmann Eitle

(57) **Abstract**

The present invention refers to a method for assessing the integrity of a circular DNA molecule in a biological sample by using digital polymerase chain reaction (dPCR). The assessment of the integrity of the circular DNA, which is carried out according to the present invention, would allow the identification of mitochondrial damage or mitochondrial affectation which may trigger the process of apoptosis or programmed cell death and consequently lead to cell death and associated pathologies.

## Description

### FIELD OF THE INVENTION

The present invention refers to the medical field. Particularly, it refers to a method for assessing the integrity of a circular DNA molecule in a biological sample by using digital polymerase chain reaction (dPCR). The assessment of the integrity of the circular DNA, which is carried out according to the present invention, would allow the identification of mitochondrial damage or mitochondrial affectation which may trigger the process of apoptosis or programmed cell death and consequently lead to cell death and associated pathologies.

### STATE OF THE ART

### Qualitative and quantitative polymerase chain reaction (PCR)

PCR is a common technique in molecular biology that allows the presence of a specific DNA fragment (target sequence) to be detected in an isolated DNA sample. It is about artificially obtaining millions of copies of a specific sequence, so that it can be detected, using a thermal cycler. To do this, the DNA is first denatured by heat, that is, its two complementary strands are separated, and then small single-stranded sequences (oligonucleotides) are hybridized, known as primers, one for each complementary strand, flanking the ends of the Target fragment of DNA to be amplified. This second step is the alignment step.

Subsequently, a DNA polymerase will add deoxynucleotides from the primers, synthesizing two new DNA strands. After this extension process, two copies of the target sequence between the two primers will be obtained. Multiple consecutive cycles of denaturation, primer alignment, and polymerase extension are performed, resulting in millions of copies of the specific target sequence from very small amounts of original DNA. After being stained by a fluorochrome, the large number of copies will give enough sensitivity to obtain a visible signal after electrophoresis or other detection procedures.

Generally, the high number of cycles finally results in saturation of the amplification level, reaching end-point PCR. Consequently, this technology is mainly qualitative, allowing to establish the presence (positive PCR) or absence (negative PCR) of the target sequence in the sample. However, if it is positive, it cannot tell us how many copies of the target sequence were initially in the sample.

Quantification of the originally present target sequences is possible using a technical variant, quantitative or real-time PCR (qPCR). In qPCR, the thermal cycler is coupled to a spectrofluorometer that measures the fluorescence emitted by a fluorochrome that binds to DNA. The more target sequence in the sample, the more PCR product there will be in each cycle of amplification. Using a fluorochrome that fluoresces when binding to DNA, the level of amplification can be monitored in each cycle. The more copies of the target, the greater the fluorescence intensity. By determining the cycle in which a significant increase in fluorescence occurs, a quantitative estimate of the target molecules initially present in the sample is obtained.

To be more precise and specific in determining the copies of the target sequence present after each cycle of amplification, an oligonucleotide probe is included that specifically hybridizes to a complementary sequence present on the target being amplified. This probe may have a fluorochrome that does not fluoresce. Once hybridized, the DNA polymerase, during the extension process, hydrolyses this probe, releasing its fluorochrome to the medium, which now emits a signal. The more copies of the amplified, the more probe hybridizes, and the more signal is detected. These are called hydrolysis probes (TaqMan).

Two different DNA targets can be quantified simultaneously, using a pair of primers that allows the amplification of one target sequence together with a pair of primers that allows the amplification of the other target sequence. One amplicon is distinguished from the other by hybridization with two TaqMan probes. One probe would be complementary to a sequence of one of the amplicons and labelled with a fluorochrome and the other would be complementary to a sequence of the other amplicon and labelled with another fluorochrome that emits at a different wavelength than the previous one.

qPCR does not allow direct absolute quantification of target sequences. It is necessary to compare the results with those obtained with a reference standard curve, performed by reactions on serial dilutions of the sample and / or an endogenous control gene.

Digital PCR (dPCR) is a new approach that allows the absolute detection and quantification of a target sequence, with greater precision and sensitivity. Digital PCR works by dividing a DNA sample into micropartitions, usually in microwells on a nanofluid chip or in microdroplets in suspension, so that there is a single or no target sequence in each partition. Each one of them behaves like a microchamber in which the end-point PCR is carried out, in isolation from the others, and is analysed individually. Consequently, instead of carrying out a single PCR on the entire sample, it is distributed in thousands of individual and parallel end-point PCR reactions.

When the DNA distributed in the microchamber contains the target sequence, it will be amplified millions of times and will generate a signal (positive partition). If there is no amplification, the DNA present in the microchamber should not have a copy of the target sequence (negative partition). During amplification, hydrolysis probes (TaqMan) labelled with a fluorochrome can be used to detect the presence of the target sequence. When there is no target sequence, no signal will accumulate. After PCR analysis, the fraction of positive reactions is used to absolutely quantify the number of target molecules in the sample, without the need for previous standard curves or endogenous controls, as was the case with qPCR. This reduces error and improves sensitivity and reproducibility. To take into account the possible microchambers that may by chance have received more than one molecule of the target sequence, a correction factor is applied using the Poisson distribution model.

### Mitochondrial DNA (mtDNA)

Mitochondria are cytoplasmic organelles of enormous importance for energy metabolism and cellular homeostasis. They generate ATP through electronic transport and oxidative phosphorylation (OXPHOS), produce reactive oxygen species (ROS), are important in lipid metabolism, calcium homeostasis, iron-sulphur complex production, and have a primary role in the initiation and execution of apoptosis process or programmed cell death. Each cell is estimated to typically contain 80-700 mitochondria, depending on the cell type. In the human oocyte they can reach up to 100,000.

Mitochondria contain their own DNA, separate from nuclear DNA. Human mitochondrial DNA (mtDNA) consists of a double-stranded DNA that forms a closed circular molecule of 16,569 base pairs (bp), located in the mitochondrial matrix. It contains 37 genes, 13 coding for proteins present in four of the five multienzyme complexes of the respiratory chain, as well as 22 coding for tRNAs and 2 for rRNAs, necessary for the synthesis of the 13 polypeptides, in mitochondrial ribosomes.

The mitochondrial genome is genetically very dense, as genes do not have introns and are contiguous or separated by one or two non-coding bases. Unlike the nuclear genome, mtDNA replicates continuously throughout the cell cycle and has a half-life of 7-10 days, depending on the cell type, reaching 31 days in the rat brain. In the fertilization process, the sperm does not contribute its mtDNA. The mtDNA of the zygote comes from the oocyte, so its inheritance is matrilineal, through the mother, although exceptional cases of biparental inheritance have been reported.

The mutation rate of mtDNA is estimated to be at least 10 times that of nuclear DNA. mtDNA is very sensitive to oxidative damage. The proximity of the respiratory chain may favour mtDNA damage by ROS generated secondarily during the OXPHOS process. It has also been suggested that the lack of associated histone proteins would confer greater deprotection on mtDNA, relative to nuclear DNA. However, mtDNA is organized into nucleoprotein complexes called nucleoids. The main associated protein is mitochondrial transcription factor A (TFAM). Each nucleoid is estimated to contain one to three mtDNA molecules and each mitochondrion may contain several nucleoids, which do not appear to exchange their mtDNA.

Ineffectiveness of mtDNA repair systems has also been suggested. But the presence of the main repair pathways has been demonstrated, including the base excision repair pathway (BER), the repair of single-strand DNA breaks (SSBs), the repair of mismatched nucleotides ("mismatch"), and the repair of double-strand DNA breaks (DSBs) by recombination or non-homologous end joining pathways, although this is not clear. The nucleotide excision repair pathway has been proven to be inoperative in mitochondria. This pathway is essential to repair injuries that cause important steric modifications in the DNA molecule, such as those caused by ultraviolet light, multiple chemical agents and toxins, which would lead to irreparable damage to the mtDNA, blocking its replication and transcription. On the other hand, if the injuries are excessive, the repair may not be efficient. In these cases, these damaged molecules can be degraded by nucleases.

However, compared to the nuclear genome, mtDNA has the advantage of its redundancy, consisting of 1,000-10,000 copies in each cell. A considerable fraction of molecules can be lost without affecting mitochondrial functions. After selective degradation of damaged molecules and depletion of mtDNA, the molecules can be replaced by mitochondrial genomic turnover, by increasing the replication of persistent intact molecules.

If the damage is excessive and the repair systems are insufficient, the damaged mitochondria can be selectively degraded through the process of mitophagy or autophagy, forming an autophagosome that fuses with a lysosome. On the other hand, mtDNA damage can trigger the process of apoptosis or programmed cell death. Thus, the dysfunction of these phenomena can lead to cell death and associated pathologies. In fact, changes in mtDNA copy number and its deletion frequency have been linked to the aging process, high blood pressure, and multiple diseases, including cancer and Alzheimer's disease.

### Common mtDNA study techniques

In the clinical genetics laboratory, the usual techniques on human mtDNA, at a clinical and research level, usually focus on determining the nucleotide sequence of specific genes, to estimate the possible presence of point mutations or deletions and duplications, in homoplasmy (all the same molecules) or in heteroplasmy (different coexisting variants), responsible for constitutional or somatic pathologies. For this, the standard techniques of Sanger sequencing or "Next Generation Sequencing" (NGS) are used.

Another common determination lies in the quantification of the copy number of mtDNA genomes, to evaluate possible depletions at the cellular level. For this, although the NGS could provide information in this regard, the previously described qPCR is usually used, amplifying a specific target sequence present in mtDNA that does not cross-react with potential homologous sequences present in nuclear DNA. As mentioned, the estimation is not simple and requires a calibration curve with serial dilutions of the mtDNA. Recently, the use of dPCR allows a direct and absolute estimation of the number of copies of mtDNA.

However, a parameter as relevant as mtDNA integrity or damage is not usually addressed in the clinical laboratory. This evaluation requires relatively complex methodologies, which makes its study more typical of the field of research. Within DNA lesions, double-strand breaks (DSBs) are considered especially harmful, leading to a breakdown in the molecule and being more difficult to repair.

It is foreseeable that the estimation and quantification of molecular damage, and especially the DSBs present in mtDNA, can provide relevant information in relation to the evaluation of the actual biological aging of the individual, of physiological stress and of a multitude of human pathologies. A DSB in the circular mtDNA genome results in its linearization. Two or more DSBs would be indicative of its fragmentation. This mtDNA fragmentation could be related to direct degradation processes by nucleases, mitophagy, autophagy or apoptosis.

Consequently, the elevation or modification of the proportion of mtDNA molecules with DSBs above the usual physiological levels could have an impact on the study of aging and associated processes, the evaluation of multiple pathologies, neoplastic, degenerative, inflammatory, genetic, etc, at the diagnostic, prognostic, follow-up and response level to various therapies.

Quantification of mtDNA DSBs has been carried out using Southern blot or qPCR techniques. The Southern blot is semi-quantitative. The mtDNA is electrophoresed under neutral conditions. The more broken, the more mtDNA fragments will migrate from the origin. The DNA is transferred to a membrane and hybridized with a labeled mtDNA probe. The intensity of hybridization is quantified with image analysis. If the DNA is broken, it results in a smear that will contain mtDNA; the more broken, the more DNA will migrate to the smear. Similarly, less mtDNA will be quantified at the origin, relative to an undamaged control in which the same number of molecules are evaluated. This is a complex, cumbersome, long and inaccurate technique. It is needed a large amount of DNA to do the study. It has low resolution and the quantification is neither precise nor absolute.

qPCR is based on the fact that multiple types of DNA lesions, including DSBs, do not allow the amplification of the molecule by the DNA polymerase. The amplification level will be lower relative to a no-damaged control DNA. It is not specific for detecting DSBs. Unlike the Southern blot, it can work with limited amounts of DNA. A control of the quantity of mtDNA molecules is necessary, which is estimated by amplifying a small segment of mtDNA, of about 300 bp, which, given its small size, is highly unlikely to contain lesions.

To have a better chance of detecting any lesion that blocks the polymerase, a long qPCR is usually performed, of almost the entire mitochondrial genome. But the efficiency of this amplification is lower in itself, due to the processivity of the polymerase and the fact that the fluorochrome itself that is used to stain DNA inhibits replication. The semi-long qPCR ameliorates these problems but has very low sensitivity. On the other hand, the reproducibility of these qPCRs is also usually low.

In conclusion, known methodologies are very complex, with significant logistical and sensitivity problems. They are not applicable for routine clinical studies.

So, there is a need of finding simpler and more effective strategies for assessing the integrity of a circular DNA.

The present invention is focused on solving this problem and a simple and effective methodology is herein presented that, in addition to being able to estimate in an absolute way the number of copies of mtDNA as previously described, it is able to quantify the molecules with DSBs.

### DESCRIPTION OF THE INVENTION

### Brief description of the invention

As explained above, the present invention refers to a method (hereinafter method of the invention) for assessing the integrity of a circular DNA molecule in a biological sample by using dPCR. The assessment of the integrity of the circular DNA, which is carried out according to the present invention, would allow the identification of mitochondrial damage or mitochondrial affectation which may trigger the process of apoptosis or programmed cell death and consequently lead to cell death and associated pathologies.

The method of the invention is based on the concept of "break-apart". The fluorescent *in situ* hybridization (FISH) technique works at the cellular scale, using the fluorescence microscope. The cell's DNA is denatured *in situ* and probes labelled with fluorochromes that recognize the complementary sequence are hybridized on it, detecting its presence and its location in the cell nucleus. One of its applications is the detection of chromosomal rearrangements that take place between two specific regions of the genome and the quantification of the number of cells that carry this rearrangement.

In one of the variants, two probes labelled with different fluorochromes are used, for example, one red and the other green, which hybridize in two neighbouring sequences, flanking the region where DNA breakage usually takes place, which then joins with another sequence in another chromosomal location. If rearrangement does not take place, the cell nuclei show the contiguous red and green signal. If reordering occurs, the red and green signals are separated. This is a "break-apart" probe. The mtDNA is too small for this FISH technique with "break-apart" probes to be applied with enough sensitivity.

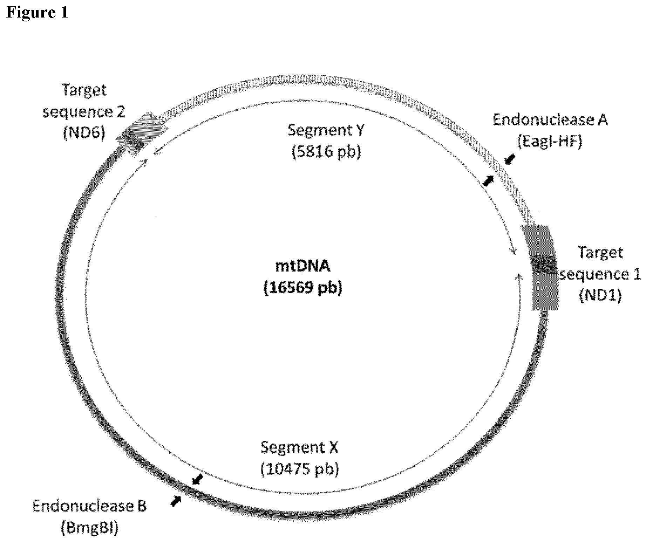

Particularly, a 16,569 bp circular human mtDNA molecule is depicted in **Figure 1****.** According to the present invention, the detection of molecules with and without DSBs is possible through the joint amplification of at least two different small target sequences (*Target Sequence 1* and *Target Sequence 2*), distant in the circular mtDNA genome, in thousands of parallel PCR reactions in independent microchambers, according to the dPCR technique.

As an illustrative example, *Target Sequence 1* may be located within the ND1 gene comprising bases 3,629 to 3,775 and *Target Sequence 2* is found within the ND6 gene comprising bases 14,250 to 14,382 **(****Figure 1****).** The small size of the two amplicons (*Target Sequence 1:* 146 bp; *Target Sequence 2*: 132 bp), makes it extremely unlikely that spontaneous DSBs coincide within any of the targets. According to this example, the two targets are connected by two segments: the longer *Segment X* of 10,475 bp and the shorter *Segment Y* of 5,816 bp **(****Figure 1****).** The longer segment is approximately twice the short segment (1.8 times). Since the two DNA segments between the two targets are very long, if there are DSBs, they will be located almost absolutely in these regions.

Amplification of *Target Sequence 1* may be detected by hybridization with a hydrolysis probe (TaqMan) labeled with a fluorochrome (for example, HEX: emission peak at 556 nm; green towards yellow), while amplification of *Target Sequence 2* may be detected with another probe labeled with a different fluorochrome (for example, FAM: emission peak at 517 nm; green towards blue). The mtDNA molecules will be in a concentration such that each microchamber contains one or none. Since mtDNA is a circular molecule, the joint amplification of both targets, that is, the presence of the signals of both fluorochromes (HEX + FAM) colocalized in a microchamber, will correspond to an intact circular molecule or with a single DSB, that is, linearized **(****Figure 2AB** " Aliquot not RE-digested").

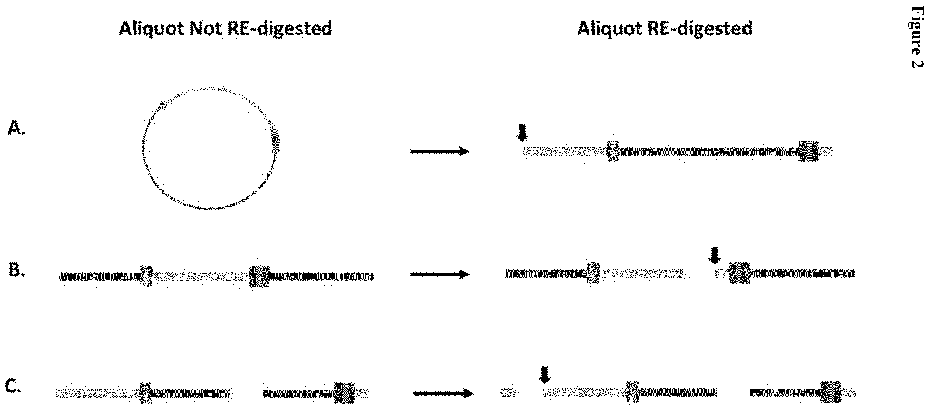

If the molecule has at least two DSBs, one in a segment between the two targets (*Segment X*) and another in the opposite segment between them (*Segment Y*), each target will be located in a different fragment, so they can be separated and be distributed in different microchambers ("break-apart"). As the PCR reaction takes place, there will be a chamber with a single fluorochrome signal (HEX) and another chamber with the unique signal of the other fluorochrome (FAM) **(****Figure 2C****,** "Aliquot Not RE-digested").

As with all quantitative studies with dPCR, this assay must be carried out ensuring an adequate proportion of microchambers without signal, to avoid as far as possible the colocalization of both targets that were separated due to DSBs, but which may coincide in the same microchamber by random. Under these conditions, the Poisson distribution allows its estimation and the corresponding correction.

As indicated, the colocalization of both signals can correspond to intact circular molecules or to linearized molecules due to a DSB, evidently located in a segment between both targets. In order to evaluate and distinguish these two possibilities, in a dPCR reaction an aliquot of the sample is previously digested with a *restriction endonuclease A* that produces a single DSB in a segment between the targets (*Segment Y*)*.* In our example, the EagI-HF endonuclease is used, which produces a single DSB in mtDNA, at bp 2,566 **(****Figure 1****).** In another dPCR reaction, another aliquot of the sample is pre-incubated with another *restriction endonuclease B* that induces a single DSB in the segment opposite to the previous one (*Segment X*). Preferably the endonuclease BmgBI is used, which produces a single DSB in mtDNA, at bp 9,827 **(****Figure 1****).** When proceeding to dPCR, if the molecule is intact, circular, the induced DSB will linearize the molecule, but will not separate the targets and the signals, the two colors, will colocalize in the microchamber (HEX + FAM) **(****Figure 2A****,** "Aliquot RE-digested"). But if the molecule already has a previous DSB and therefore is already linearized before digestion, one of the enzymes will be able to produce another DSB in the intact segment between the two, so the targets can be separated into different chambers, which will give signal of a single color, different between the two **(****Figure 2B****,** "Aliquot RE-digested").

Through digestion-linearization with a *restriction endonuclease A,* the frequency of molecules with an original DSB located in one of the segments between both targets (*Segment X*) is evaluated. On the other hand, digestion-linearization with the other *restriction endonuclease B* makes it possible to estimate the proportion of molecules with a DSB located in the segment between both targets, opposite to the previous one (*Segment Y*). The digestion by any of the endonucleases, of originally fragmented mtDNA molecules, would not modify the proportion of microchambers with separate signals obtained in the aliquot of the sample that has not been digested **(****Figure 2C****,** "Aliquot Not RE-digested" and "Aliquot RE-digested").

The technique detects the presence of two or more DSBs flanking the two targets, located in the opposite DNA segments between them. As a possible limitation, the presence of two or more original DSBs that do not flank the two targets, that is, in a single segment between them, will not be detected in the undigested sample, as the targets are not physically separated, keeping the signals colocalized. The molecule would be shorter and linearized, with both targets connected by the opposite segment. The endonuclease cleavage in this connecting segment would lead to the separation of the targets. But it cannot be discerned whether there was originally a single DSB or more previous DSBs in the first segment. However, if the DSBs are generated stochastically, given the distance of the two targets, the probability of this fact is very low, especially if the basal levels are low, as is usually the case at the biological level (see **Example 2).** In addition, it must be taken into account that the detection of two DSBs in native mtDNA is considered indicative of the fragmentation or degradation of the mtDNA, so the presence of multiple dispersed breaks throughout the molecule is foreseeable, resulting in physical separation of both targets.

In summary, in a physiological situation, in the case of the presence of DSBs in the mtDNA, they are expected to be distributed in two types of molecules: (a) A subpopulation of fragmented molecules, possible consequence of nuclease degradation, typical of mitochondrial dynamics, or in relation to processes of mitophagy, autophagy or apoptosis or another form of cell death. For example, the half-life of rat mtDNA has been estimated at 6.7 days in the heart, 9.4 days in the liver, 10.4 days in the kidney, and over 31 days in the brain; and (b) Another subpopulation of molecules with DSBs resulting from random damage. This subpopulation must be very limited and the DSBs would be scattered throughout the global mtDNA population. Then if the molecule has DSBs, this would be unique, being very unlikely that two or more DSBs coincided in the same molecule. In our assay, the subpopulation of fragmented molecules (a) would be estimated by dPCR in undigested DNA. The subpopulation of molecules with presumed random DSBs (b), initially linearized, would be recognized by dPCR after digestion with the endonuclease.

Thus, the "break-apart" assay is based on the comparison of the results of the dPCR with an aliquot of the undigested sample and the dPCR of another aliquot of the sample previously digested with a *restriction endonuclease A* and the dPCR of another aliquot of the sample previously digested with the other *restriction endonuclease B.* In this way, the frequency (%) of three populations of mtDNA molecules present in a sample can be estimated **(****Figure 2****):**
1. Originally fragmented DNA with at least two DSBs between the targets, each DSB being located in each segment between the two targets. Preferably, the fraction of originally fragmented DNA molecules with at least two DSBs, one in each segment between the two targets, is identified by determining the percentage of microchambers showing one of the two signals corresponding to one of the target sequences in an aliquot which has not been digested by endonuclease enzymes.
2. Originally linear DNA with a least one DSB between the targets. Preferably, the fraction of originally linear DNA molecules with at least one DSB between the two targets, is identified by implementing the following formula: [(percentage of microchambers showing one of the two signals corresponding to one of the target sequences in an aliquot which has been digested by the first endonuclease enzyme) - (percentage of microchambers showing one of the two signals corresponding to one of the target sequences in an aliquot which has not been digested by endonuclease enzymes)] + [(percentage of microchambers showing one of the two signals corresponding to one of the target sequences in an aliquot which has been digested by the second endonuclease enzyme) - (percentage of microchambers showing one of the two signals corresponding to one of the target sequences in an aliquot which has not been digested by endonuclease enzymes)].

The molecules that are already fragmented in origin (with separate signals) would remain the same in the aliquots digested with the endonucleases. The extra molecules with separate signals, obtained after digestion with an endonuclease, will correspond to linearized molecules, with a DSB in origin. The first term of the sum estimates the population of molecules with a DSB in origin in one of the segments between the targets (*segment X*) and the second term, in the opposite segment (*segment Y*). If the DSBs are randomly induced and the targets are equidistant on the mtDNA molecule, that is, the two segments are equal in length, it would be possible to perform a single digestion and multiply its results by 2.

If the segments are unequal, the probability of a DSB in the small segment could be estimated according to the proportion corresponding to that found in the large segment, after digestion with the relevant endonuclease. However, for greater safety, it is preferable to study both segments separately, using two endonucleases.

3. Originally orthodox circular DNA without DSB. Preferably, the fraction of originally orthodox circular DNA molecules without any DSB is identified by implementing the following formula: [100% - (percentage of originally fragmented DNA molecules + percentage of originally linear DNA molecules)].

As referred to item 2 above, the molecules with a single DSB are estimated by subtracting the molecules with fragmentation (2 or more DSBs) obtained in the undigested aliquot from those obtained in the digested aliquot. The difference would correspond to molecules with an original, linearized DSB, which the enzyme has cut giving rise to two DSBs, allowing the separation of the targets.

We can consider a situation where the two targets were equidistant on the mtDNA molecule and the unique DSB is randomly distributed into the two equally sized segments (*X segment = Y segment*), which separate them. If the frequency of molecules with a single DSB is very low, the increase in molecules with separate targets after digesting with an endonuclease that estimates whether the DSB occurred in one of the *segment X* will be very small, resulting in a figure close to the of the undigested aliquot. The same will happen after digesting with the other endonuclease, to study the other *segment Y.* Then it can happen that neither of the two increases with respect to the molecules that already had fragmentation, reaches statistical significance. We cannot estimate their presence confidently.

But if the targets are located asymmetrically in the mtDNA molecule (*segment X > segment Y*), the larger *segment X* between the targets will have a higher probability of presenting an original DSB, even though the other smaller *segment Y* has lower probability. If this situation is forced and both targets are placed too close in the mtDNA molecule, the possibility of an original DSB occurring in this minimal segment, separating them, can be practically nil, but much higher in the large segment. Logistically, this minimum segment could be used as a site of endonuclease cleavage, with each target sequence being located at one end of the linear molecule, leaving almost the entire length of the mtDNA as a segment to show a DSB. The probability that this DSB would be detected would be the highest. But this design has a much higher risk of difficulty in recognizing whether the separation of the targets has been by an original DSB or by basal fragmentation.

A reasonable option for the selection of the two targets is that they be located in the mtDNA molecule, in such a way that a segment that connects them on one side is approximately twice the length of the one that connects them on the other (*segment X = 2 segment Y*). Given the habitual global low levels of DSBs, this allows that at least one segment, the largest, can contain a single DSB with a greater chance of giving a statistically significant result; and maintaining the ability to differentiate fragmentation from the single DSB. In our practical example design, the size of *segment X* is almost twice (1.8 times) that of *segment Y.*

The technical design is applicable not only to mtDNA but to any circular DNA, such as plasmids, or linear DNA fragments that can be circularized by ligation of the ends.

Although a design based on the evaluation of two target sequences is herein presented, it is possible to increase the number of target sequences distributed in the molecule, performing a multiplex dPCR, provided that the amplification of each one is recognizable with a specific probe for it, whose fluorochrome or signal is different from the others, and identifiable by the scanner. Depending on the pattern of signals that coexist or not in each microchamber, the presence of a DSB and its location in a narrower segment delimited between two of the targets could be recognized.

So, the first embodiment of the present invention refers to an in *vitro* method for assessing the integrity of a circular DNA molecule in a biological sample by amplifying and detecting specific target sequences, which comprises:
a) Preparing at least the following three aliquots:
   i. Aliquot comprising the circular DNA to be analysed,
   ii. Aliquot comprising the circular DNA to be analysed along with a first endonuclease enzyme which cleaves one of the two DNA segments of the circular DNA molecule which is located between two specific target sequences to be detected, and
   iii. Aliquot comprising the circular DNA to be analysed along with a second endonuclease enzyme which cleaves the other DNA segment of the circular DNA molecule which is located between the two specific target sequences to be detected;
b) Performing digital polymerase chain reaction (dPCR) on each of the aliquots prepared according to the step a) by amplifying and detecting the specific target sequences of the circular DNA molecule with different fluorochromes or signals;
c) Assessing the integrity of the circular DNA by identifying the following fractions of DNA which may be present in each of the aliquots prepared according to the step a):
   i. Originally fragmented DNA with at least two double-strand breaks (DSBs) between the targets, each DSB being located in each segment between the two targets;
   ii. Originally linear DNA, with a least one DSB between the targets;
   iii. Originally orthodox circular DNA without DSB;
wherein the presence of originally fragmented DNA molecules and/or originally linear DNA molecules is an indication of DNA damage of the circular DNA and the presence of originally orthodox circular DNA molecules is an indication of DNA integrity.

In a preferred embodiment, the method of the invention is characterised in that:
- The fraction of originally fragmented DNA molecules with at least two DSBs, one in each segment between the two targets, is identified by determining the percentage of microchambers showing one of the two signals corresponding to one of the target sequences in an aliquot which has not been digested by endonuclease enzymes;
- The fraction of originally linear DNA molecules with at least one DSB between the two targets, is identified by implementing the following formula: [(percentage of microchambers showing one of the two signals corresponding to one of the target sequences in an aliquot which has been digested by the first endonuclease enzyme) - (percentage of microchambers showing one of the two signals corresponding to one of the target sequences in an aliquot which has not been digested by endonuclease enzymes)] + [(percentage of microchambers showing one of the two signals corresponding to one of the target sequences in an aliquot which has been digested by the second endonuclease enzyme) - (percentage of microchambers showing one of the two signals corresponding to one of the target sequences in an aliquot which has not been digested by endonuclease enzymes)]; and
- The fraction of originally orthodox circular DNA molecules without any DSB is identified by implementing the following formula: [100% - (percentage of originally fragmented DNA molecules + percentage of originally linear DNA molecules)].

In a preferred embodiment, the circular DNA molecule is mitochondrial DNA. In a preferred embodiment, the target sequences can be located at different physical distances in the circular DNA molecule, from contiguous to equidistant.

In a preferred embodiment, the method is performed by detecting more than two target sequences and wherein the amplification of each one would be detected with a specific probe labelled with a fluorochrome or signal different from that of the others.

In a preferred embodiment, the integrity of the circular DNA molecule is assessed by determining the presence or absence of DSBs.

The second embodiment of the present invention refers to a method for identification of mitochondrial damage or mitochondrial affectation which comprises carrying out the method of the invention described above, wherein the identification and quantification of fragmented and/or linearized mitochondrial DNA, or abnormal level of orthodox circular mitochondrial DNA without DSBs, is an indication of mitochondrial damage or mitochondrial affectation.

In a preferred embodiment, the mitochondrial damage or mitochondrial affectation is a consequence of mitochondrial DNA mutations or depletion, or due to dysfunction of nuclear genes involved in mitochondria structure and physiology, or associated to diseases like diabetes, Huntington's disease, cancer, Alzheimer's disease, Parkinson's disease, bipolar disorder, schizophrenia, aging and senescence, anxiety disorders, cardiovascular disease, sarcopenia, chronic fatigue syndrome, or due to the effect of drugs, physical, chemical or biological agents, or to other conditions that may affect health.

The third embodiment of the present invention refers to an aliquot preparation kit, or a kit-of-parts, comprising:
a) Aliquot comprising the circular DNA;
b) Aliquot comprising the circular DNA to be analysed along with a first endonuclease enzyme which cleaves one of the two DNA segments of the circular DNA molecule which is located between the at least two specific target sequences to be detected, and
c) Aliquot comprising the circular DNA to be analysed along with a second endonuclease enzyme which cleaves the other DNA segment of the circular DNA molecule which located between the at least two specific target sequences to be detected.

The fourth embodiment of the present invention refers to a dPCR kit comprising an aliquot preparation kit, or a kit-of-parts, which in turns comprises:
a) Aliquot comprising the circular DNA;
b) Aliquot comprising the circular DNA to be analysed along with a first endonuclease enzyme which cleaves one of the two DNA segments of the circular DNA molecule which is located between the at least two specific target sequences to be detected, and
c) Aliquot comprising the circular DNA to be analysed along with a second endonuclease enzyme which cleaves the other DNA segment of the circular DNA molecule which located between the at least two specific target sequences to be detected.

The fifth embodiment of the present invention refers to the use of the above described dPCR kit for assessing the integrity of a circular DNA molecule.

For the purpose of the present invention the following terms are defied:
- The term "comprising" means including, but not limited to, whatever follows the word "comprising". Thus, use of the term "comprising" indicates that the listed elements are required or mandatory, but that other elements are optional and may or may not be present.
- By "consisting of' it is meant including, and limited to, whatever follows the phrase "consisting of'. Thus, the phrase "consisting of' indicates that the listed elements are required or mandatory, and that no other elements may be present.

### Description of the figures

**Figure 1****. Scheme of the experimental model for the detection of double-strand DNA breaks (DSBs) in the mitochondrial DNA molecule (mtDNA) by digital PCR (dPCR).** The human mtDNA molecule is a 16,569 bp circle. The detection of molecules with and without DSBs is possible through the joint amplification of two different small target sequences (*Target Sequence 1* and *Target Sequence 2*), distant in the circular mtDNA genome, in thousands of parallel PCR reactions in independent microchambers, according to the dPCR technique. In our practical design, the 146 bp *Target Sequence 1* is located within the ND1 gene and its amplification is detected with a fluorochrome HEX-labeled hydrolysis probe. *Target Sequence 2*, 132 bp, is found within the ND6 gene and its amplification is detected with a hydrolysis probe labeled with fluorochrome FAM. The two targets are connected by two segments: the longer *Segment X,* of 10,475 bp, and the shorter *Segment Y,* of 5,816 bp. The *restriction endonuclease (A),* EagI-HF, produces a single DSB in *Segment Y* (arrow). The *restriction endonuclease (B),* BmgBI, induces a single DSB in *Segment X* (arrow).

**Figure 2****. Scheme of possible results obtained by the experimental model to detect mtDNA without and with DSBs.** For the assay, the DNA sample is divided into three tubes. One contains an undigested aliquot. Another contains an aliquot digested by an endonuclease A that induces a DSB in a connecting segment between the two target sequences (for example, EagI-HF, which cuts in the Y segment, transforming a circular mtDNA molecule into a linear molecule with both targets joined by segment X). The latter contains an aliquot digested by another endonuclease B that induces a DSB in the other connecting segment between the two target sequences (for example, BmgBI, which cuts in the X segment, transforming a circular mtDNA molecule into a linear molecule with both targets joined by segment Y). In the scheme, the two target sequences are shown as boxes of different color. Endonuclease cleavage is indicated by an arrow. **A.** After dPCR, a circular mtDNA molecule originally without DSBs will give two colocalized signals (FAM + HEX) in the same microchamber, in the sample not digested by the endonuclease (left). If it is digested with any of the endonucleases, a DSB will be generated in a connecting segment between both target sequences, linearizing the molecule. Both targets will remain connected by the other opposite segment, so the signals will be co-located (FAM + HEX) (right). **B.** If the mtDNA molecule originally has a DSB in a connecting segment between the two targets, it will be linearized in origin. In the undigested aliquot, the two signals will be colocalized (FAM + HEX), as in the circular molecule (left). In that aliquot digested by the endonuclease that induces a DSB in the initially intact connecting segment, the break will give rise to two separate fragments, each one with a target, therefore the signals will be distributed in different microchambers (right). **C.** If the mtDNA molecule is originally fragmented, both targets will be separated in the undigested aliquot (left) and also in the aliquots digested by one or the other endonuclease (right).

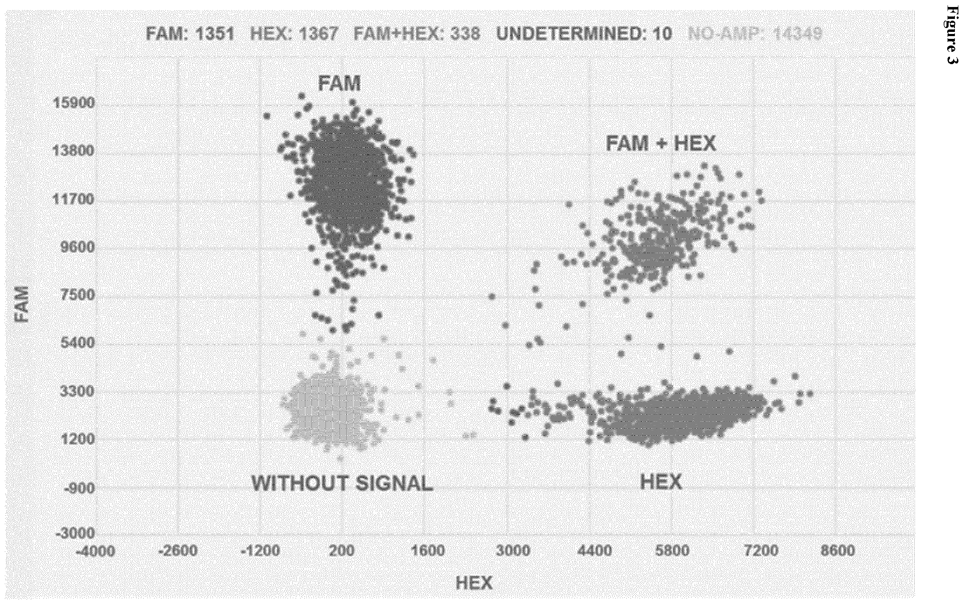

**Figure 3****. Graph of quantification and distribution of the number of microwells of dPCR chips, without and with amplification signals of the mtDNA targets.** A standard dPCR plot is shown above, the X axis of which corresponds to the HEX signal intensity and the Y axis to the FAM signal intensity. Each microwell on the dPCR chip is represented between those axes. In this graph, the upper right shows the positive microchambers for both FAM + HEX signals (green dots). At the top left, the microwells with a single FAM signal (points in blue) and at the bottom right, those showing only a HEX signal (points in red). Negative wells are depicted at the bottom left, with no signal of any fluorochrome (yellow dots).

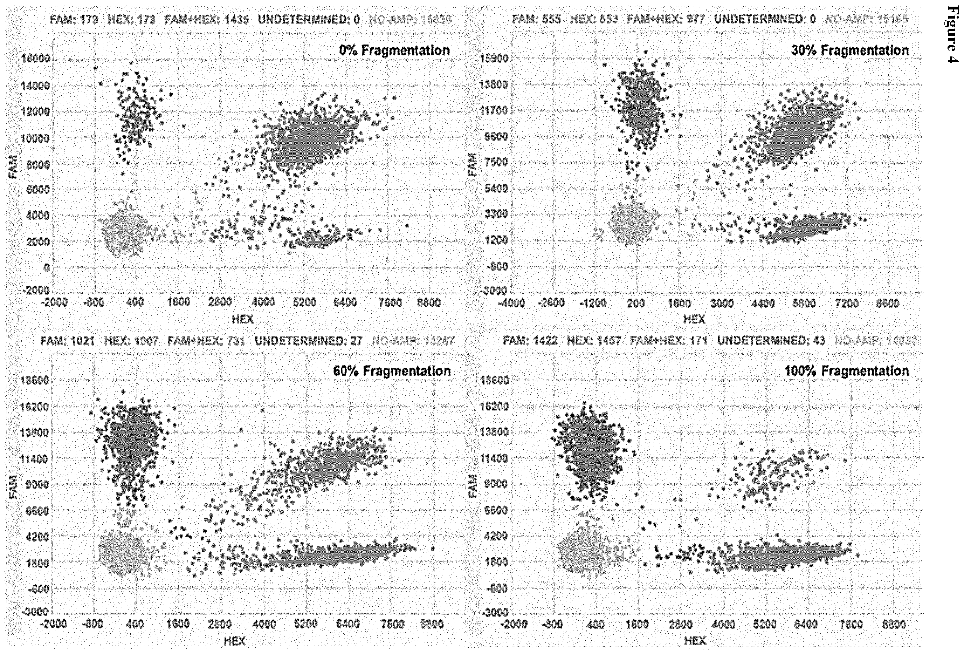

**Figure 4****. Four dPCR graphs corresponding to different percentages of mtDNA molecules with fragmentation induced by two endonucleases, EagI-HF and BmgBI: 0, 30, 60 and 100%.** These percentages were theoretically estimated, uncorrected for baseline level. As fragmentation increases, the partitions with double signal FAM + HEX (points in green) decrease and the partitions with single signal FAM (points in blue) and HEX (points in red) increase.

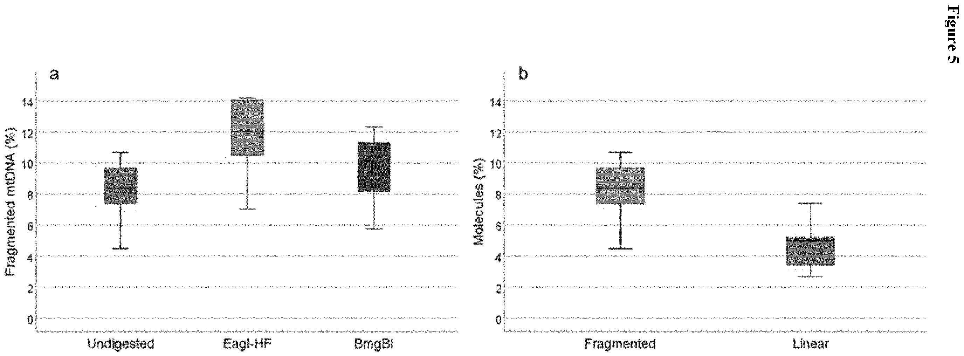

**Figure 5****. Representation of the statistics of the percentage of mtDNA fragmented and linearized in origin, obtained in 10 cell blood samples.** The data are presented as box and whisker plots. The horizontal line in the box indicates the median, the lower line of the box is the first quartile, the upper line of the box is the third quartile, and the whiskers (the end of the vertical lines) are maximum and minimum values. **A.** Undigested, EagI-HF digested and BmgBI digested aliquots. EagI-HF and BmgBI increased the fraction of molecules with separated targets with respect to that from the respective undigested aliquots. The increase was more significant for EagI-HF digested aliquots. **B.** % fragmented and % linear molecules. The percentage of linear molecules was lower than that of fragmented.

### Detailed description of the invention

The present invention is illustrated by means of the Examples set below without the intention of limiting its scope of protection.

### Example 1. Demonstration of the capacity of the "break-apart" assay by dPCR for the detection and quantification of the proportion of fragmented mtDNA

The equipment and reagents used were the following:
- Thermal cycler: QuantStudio ^{™} 3D Digital PCR Systems. ThermoFisher.
- Chips: A26316: QuantStudio ^{™} 3D Digital PCR 20K Chip Kit v2. ThermoFisher Scientific.
- Master mix: A26358 QuantStudio ^{™} 3D Digital PCR Master Mix v2. ThermoFisher Scientific.
- Water: 03315959001 Water PCR grade. Roche Diagnostics GmbH.
- Primers:
   - ND1. 10031255 ddPCR ^{™} Gene Expression Assay: MT-ND1, human. Bio-Rad.
   - ND6. 10031252 ddPCR ^{™} Gene Expression Assay MT-ND6, human. Bio-Rad.
- Restriction enzymes:
   - EagI-HF^{®} # R3505S. New England BioLabs Inc.
   - BmgBI # R0628S. New England BioLabs Inc.

This experiment was performed using the experimental design presented in **Figures 1** and **2****.**

A sample of DNA isolated from peripheral blood was distributed into three tubes:
- Tube A: The DNA (and enclosed mtDNA) remained undigested.
- Tube B: The DNA was digested with EagI-HF, which induces a DSB in the mtDNA, giving rise to linearized mtDNA molecules.
- Tube C: The DNA was digested with two endonucleases simultaneously: EagI-HF + BmgBI, resulting in two DSBs, so that the targets were in separate fragments of mtDNA.

Different mixtures of DNA from tube B (containing linearized mtDNA) with DNA from tube C (containing fragmented mtDNA) were made, obtaining tubes with theoretically 0, 10, 20, 30, 50, 60, 70, 80, 90 and 100% fragmented mtDNA **(Table 1:** Fragmentation-theoretical). From each of these tubes, 2 µl containing 0.1 ng DNA were obtained, which were mixed with 7.5 µl of Master Mix (ThermoFisher Scientific), 0.75 µl of primer solution and hydrolysis probe for target sequence 1, HEX-labeled, 0.75 µl of primer solution and hydrolysis probe for target sequence 2, FAM-labeled and 4 µl of water. These 15 µl were distributed on a dPCR chip.

dPCR was performed with a QuantStudio 3D Digital PCR System. The PCR conditions were: one cycle of 96 °C, 10 min; 39 cycles of hybridization-extension at 55°C, 1 min and denaturation at 98°C, 30 s, and a final cycle of 60°C, 2 min. After dPCR, the number of wells without any signal, with FAM + HEX signal, with single FAM signal and with single HEX signal was estimated. **Figures 3** and **4** show some layout diagrams of these wells. The analysis software made the pertinent corrections and presented the results.

After correcting for baseline value of fragmentation (11.29%), and its influence on each of the mixtures, the fragmentation frequencies obtained by evaluating the chips reproduced the theoretically expected levels in each tube, perfectly **(Table 1:** Fragmentation-dPCR and Fragmentation-theoretical-corrected) (Pearson's r: 0.999, p<0.001; linear regression y=(0.89 + 1.08)+(0.99 ± 0.02)x, R²=0.997).

**Table 1**

| **Fragmentation - theoretical (%)** | **FAM real (molec/µl)** | **Fragmentation - dPCR (%)** | **Fragmentation - theoretical - corrected (%)** |
|---|---|---|---|
| Without RE digestion | 126.93 | 8.85 | |
| 0 | 120.13 | 11.29 | 11.29 |
| 10 | 129.72 | 21.65 | 20.27 |
| 20 | 131.86 | 29.2 | 29.25 |
| 30 | 123.26 | 38.54 | 38.23 |
| 50 | 163.01 | 59.25 | 56.18 |
| 60 | 143.85 | 62.95 | 65.16 |
| 70 | 119.79 | 76.03 | 74.14 |
| 80 | 291.87 | 80.83 | 83.12 |
| 90 | 135.29 | 91.46 | 92.10 |
| 100 | 129.72 | 101.08 | 101.08 |

### Results of the experiment of quantification of the percentage of fragmented mtDNA molecules present in the sample.

The result confirms that the assay allows, in addition to estimating mtDNA copies, accurately quantifying the proportion of molecules with two or more DSBs, i.e, fragmented. Moreover, evidences the possibility of detection of a unique DSB, which linearizes the mtDNA molecule. In this case, the unique DSB had been experimentally generated by one endonuclease. The use of the other endonuclease transformed the linear molecule, with both signals together in the same well, into fragmented, with the two signals separated, in different microwells.

### Example 2. Example of quantification of mtDNA molecules without detectable DSBs, with one DSB (linearized), and with two or more DSBs (fragmentation)

To evaluate the variability of the assay, the fraction of mtDNA molecules with separated targets was quantified in five different dPCR chips for each of the three aliquots from one sample (sample 1), i.e undigested, EagI-HF digested and Bmg-BI digested. The results obtained were, for the undigested: 7.97 + 0.39, mean + sd; for EagI-HF: 12.59 + 0.53; and for Bmg-BI: 10.73 + 0.64. Coefficients of variation were 4.94, 4.20 and 5.93, respectively.

Ten different samples of DNA isolated from human peripheral blood cells were studied. Each sample was divided into three tubes:
- Tube A: The DNA remained undigested, including the mtDNA present in it.
- Tube B: The DNA was digested with EagI-HF, which induces a DSB in the mtDNA at the Y segment between both target sequences, giving rise to linearized mtDNA molecules. The targets would remain connected by the longest X segment.
- Tube C: The DNA was digested with BmgBI, which induces a DSB in the mtDNA present in the sample aliquot, at the X segment level between both targets, giving rise to linearized mtDNA molecules. The targets would remain connected through the shorter Y segment.

After dPCR, three classes of mtDNA, (1) fragmented, (2) linearized and (3) without detectable DSBs, were estimated.
(1) The fraction of original fragmented mtDNA molecules corresponded to that observed in the undigested aliquot; e.g. in sample 1: 7.97%.
(2) The fraction of linear molecules in origin will correspond to those that have at least one DSB located in the long segment X (2a; targets will be separated by EagI-HF resulting in an increase in wells with a single signal, with respect to the present in the undigested aliquot) and those that have it located in the short segment Y (2b; targets will be separated by BmgBI). In sample 1, 2a: the fraction of molecules that had at least one DSB in segment X will be: 12.59% - 7.97% = 4.62%;
   2b: In case of segment Y, it will be: 10.73% - 7.97% = 2.76%.

The fraction of initially linearized mtDNA molecules will be 2a + 2b: 4.62% + 2.76% = 7.38%. (3) The fraction of molecules without detectable DSBs will be in sample 1: 100% - (7.97% + 7.38) = 83.11%.

The results of the 10 samples are presented in **Figure 5****.** EagI-HF and BmgBI increased the fraction of molecules with separated targets with respect to that from the respective undigested aliquots (Friedman test, p<0.001). The increase was significant for EagI-HF (p<0.001) but close to significance for BmgBI (p=0.076) **(****Figure 5a****).** The proportion of initially linear molecules was significantly lower than that of fragmented (median: 5.01 versus 8.38, respectively; Wilcoxon test, p=0.007; **Figure 5b****).**

Three facts can be deduced from the results of this experiment:
1) After respective RE digestion, the long segment X yielded a higher fraction of molecules with separate targets than the short segment Y (median: 3.34 versus 1.50; Wilcoxon test, p=0.005). The X segment which is 1.8 times longer than the Y segment yields 2.2 times higher fraction of linearized molecules. Thus, the fraction of initially linearized molecules tended to be proportional to the size of the segment connecting the targets. It suggests that the DSBs were randomly distributed in the molecule and in the global population of mtDNA molecules. Taking this into account and given the very low frequency of this type of molecules, it is most likely that the linearization is due to a single DSB in a connector segment. From a probabilistic point of view, there should be a much higher fraction of molecules of this type, for some of them to have two or more DSBs in the same segment.
2) A design where the two targets are adjacently located in the molecule could have more chance to detect a unique DSB in the much bigger connector segment, but it could be difficult to be distinguished from fragmentation by two or more DSBs. Otherwise, if both targets were equidistant, the probability of presence of a DSB must be equally distributed between both connection segments, so the increase of separated targets after each RE digestion could be more difficult to achieve significance. The asymmetrical design, with a segment near twice the size of the short segment, allows that, at least, the largest segment can contain a single DSB with a greater chance of giving a statistically significant result; and maintaining the ability to differentiate fragmentation from the single DSB.
3) The frequency of mtDNA molecules originally linear by a DSB was never higher than the frequency of molecules that have two or more DSBs flanking both targets. This fact supports that the observed fragmentation could be mainly of biological origin and not mechanical due to the extraction and manipulation process of the sample. If the fragmentation were of mechanical cause, since mechanical DSBs are induced randomly in the entire population of mtDNA molecules, the fraction of molecules with a single DSB should be found much larger than the fraction with two or more DSBs, which was not the case.

### Example 3. Comparison of the frequency of mtDNA molecules with DSBs in nuclear and extracellular DNA

Monolayer cultures of the human osteosarcoma cell line U2OS were used. Cultures were finished when a cell confluence of 60% and 80% was reached. DNA was extracted from cells and their extracellular medium, separately. To eliminate residues from the culture medium, it was centrifuged at 2500 rpm for 10 minutes and the clean supernatant was collected. The assay was performed as in the previous experiment

The results are presented in **Table 2.** A slight increase of mtDNA fragmented and linearized was evidenced in cultures at 80% confluence of monolayer in comparison to 60% confluence. Extracellular mtDNA showed a much higher fragmentation, around 40%. In this case, the RE-digestions did not increase the frequency of separated targets with respect to undigested DNA, corresponding to linearized molecules. This suggests that that the damage by DSBs in the cell free mtDNA, when present, corresponds practically in its entirety to fragmentation.

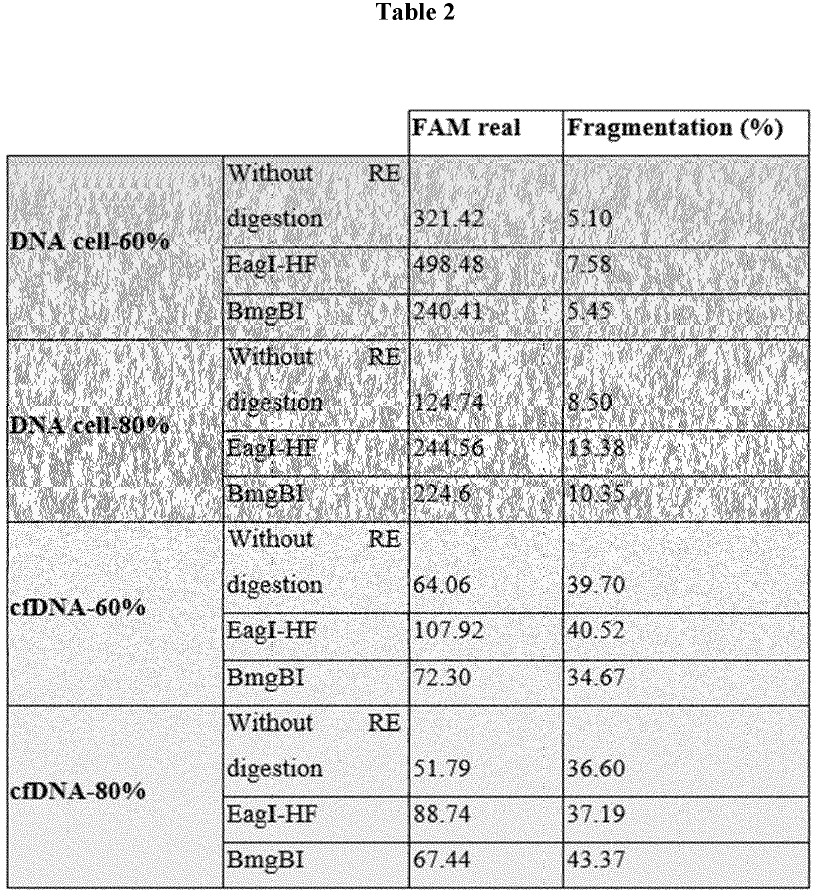

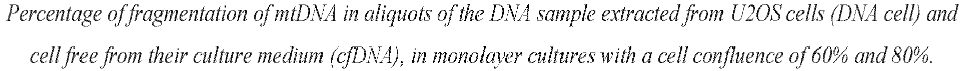

### Example 4. Evaluation of mtDNA fragmentation when depleted by ethidium bromide (EtBr) treatment

EtBr is a fluorescent dye that intercalates between base pairs of DNA. When proliferating cells are incubated with low concentrations of EtBr, it binds better to negatively supercoiled DNA substrates than on positively supercoiled ones. The mtDNA corresponds to a negatively supercoiled circular substrate, so that EtBr preferentially intercalates and inhibits its replication by mitochondrial DNA gamma polymerase. For this reason, EtBr has been used experimentally to deplete the mtDNA of cells and obtain rho zero cell lines.

Cultures of U2OS were incubated with EtBr, 500 ng/ml, for 24 h and 48 h. The number of cells initially seeded was calculated in such a way that all cultures were finished with a confluence of 80%. The cells were detached from the bottom of the flask, separated from the culture medium, washed and their DNA was extracted. The assay was performed as in the previous experiments. The results are shown in **Table 3.** EtBr drastically reduced the amount of mtDNA molecules, about 8 times after 24 h and 9 times after 48 h. In persistent molecules, an increase in the fraction with fragmentation was observed with the incubation time, from 3.73% to 14.1% after 24 h and to almost a third of the total persistent after 48 h.

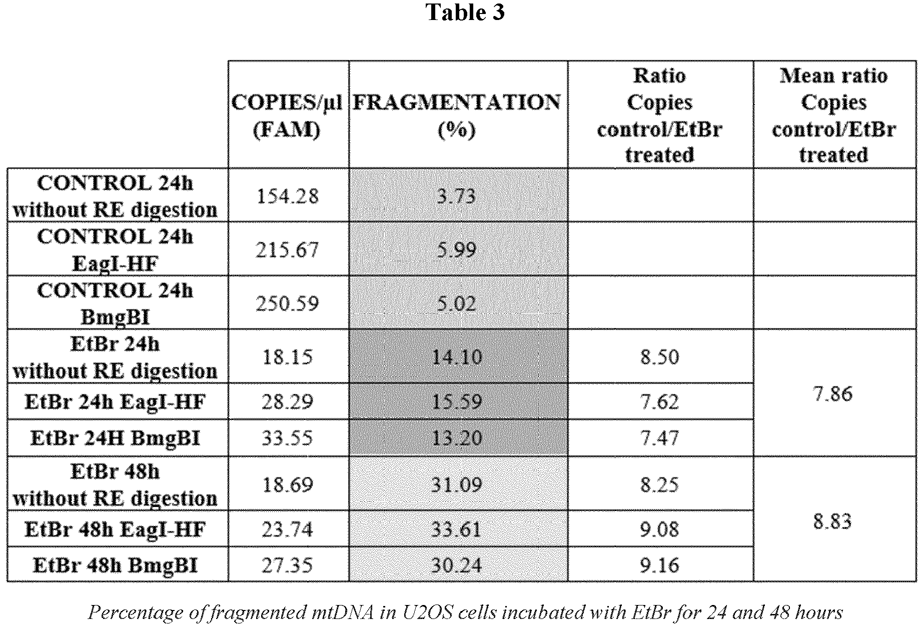

Digestion with the enzymes in the control culture showed that the frequency of linearized molecules was (5.99-3.73) + (5.02-3.73) = 3.55%, similar to that of fragmented. However, an increase in linearized molecules was not clearly demonstrated in the EtBr-treated cultures. That is, the damage observed at the level of mtDNA breaks corresponded to fragmentation, with two or more DSBs, and not to a single DSB. It is possible that mtDNA molecules blocked in their replication are eliminated by their degradation.

With common techniques for quantifying mtDNA copies, such as qPCR, the agent is observed to reduce mtDNA molecules. But the digital PCR technique shows that, in addition to depleting the mtDNA, part of the molecules that persist may be fragmented, so that the potentially functional molecules would be less than those recognized.

This result is of interest from a clinical point of view. Thus, certain drugs, such as reverse transcriptase inhibitor nucleoside analogues, which have been used for the treatment of HIV-AIDS, when incorporated into the mtDNA of cells, do not allow replication by mitochondrial DNA polymerase, decreasing its number of copies. Thus, these drugs can cause side effects as a consequence of mtDNA depletion. But these effects are variable according to the individuals. Using the technique of the present invention, it would be possible to estimate with greater fidelity the real effect of these drugs on mtDNA, not only on the number of copies but also on the integrity of the persistent ones, which would be more precisely related to the possible appearance and severity of side effects.

Regarding human health, changes in mtDNA copy number and deletion frequency have been linked to the aging process, high blood pressure, and multiple diseases, including cancer and Alzheimer's disease. The availability of a relatively simple and precise assay, based on dPCR, will allow to complement these studies and estimating the potential value of mtDNA integrity in clinical practice.

## Claims

1. *In vitro* method for assessing the integrity of a circular DNA molecule in a biological sample by amplifying and detecting specific target sequences, which comprises:
a) Preparing at least the following three aliquots:
i. Aliquot comprising the circular DNA to be analysed,
ii. Aliquot comprising the circular DNA to be analysed along with a first endonuclease enzyme which cleaves one of the two DNA segments of the circular DNA molecule which is located between two specific target sequences to be detected, and
iii. Aliquot comprising the circular DNA to be analysed along with a second endonuclease enzyme which cleaves the other DNA segment of the circular DNA molecule which is located between the two specific target sequences to be detected;
b) Performing digital polymerase chain reaction (dPCR) on each of the aliquots prepared according to the step a) by amplifying and detecting the specific target sequences of the circular DNA molecule with different fluorochromes or signals;
c) Assessing the integrity of the circular DNA by identifying the following fractions of DNA which may be present in each of the aliquots prepared according to the step a):
i. Originally fragmented DNA with at least two double-strand breaks (DSBs) between the targets, each DSB being located in each segment between the two targets;
ii. Originally linear DNA, with a least one DSB between the targets;
iii. Originally orthodox circular DNA without DSB;
wherein the presence of originally fragmented DNA molecules and/or originally linear DNA molecules is an indication of DNA damage of the circular DNA and the presence of originally orthodox circular DNA molecules is an indication of DNA integrity.

2. *In vitro* method, according to claim 1, **characterised in that**:
a) The fraction of originally fragmented DNA molecules with at least two DSBs, one in each segment between the two targets, is identified by determining the percentage of microchambers showing one of the two signals corresponding to one of the target sequences in an aliquot which has not been digested by endonuclease enzymes;
b) The fraction of originally linear DNA molecules with at least one DSB between the two targets, is identified by implementing the following formula: [(percentage of microchambers showing one of the two signals corresponding to one of the target sequences in an aliquot which has been digested by the first endonuclease enzyme) - (percentage of microchambers showing one of the two signals corresponding to one of the target sequences in an aliquot which has not been digested by endonuclease enzymes)] + [(percentage of microchambers showing one of the two signals corresponding to one of the target sequences in an aliquot which has been digested by the second endonuclease enzyme) - (percentage of microchambers showing one of the two signals corresponding to one of the target sequences in an aliquot which has not been digested by endonuclease enzymes)]; and
c) The fraction of originally orthodox circular DNA molecules without any DSB is identified by implementing the following formula: [100% - (percentage of originally fragmented DNA molecules + percentage of originally linear DNA molecules)].

3. *In vitro* method, according to any of the claims 1 or 2, wherein the circular DNA molecule is mitochondrial DNA.

4. *In vitro* method, according to any of the previous claims, wherein the target sequences can be located at different physical distances in the circular DNA molecule, from contiguous to equidistant.

5. *In vitro* method, according to any of the previous claims, wherein the method is performed by detecting more than two target sequences and wherein the amplification of each one would be detected with a specific probe labelled with a fluorochrome or signal different from that of the others.

6. *In vitro* method, according to any of the previous claims, wherein the integrity of the circular DNA molecule is assessed by determining the presence or absence of DSBs.

7. *In vitro* method for the identification of mitochondrial damage or mitochondrial affectation which comprises carrying out the method of any of the claims 1 to 6, wherein the identification and quantification of fragmented and/or linearized mitochondrial DNA, or abnormal level of orthodox circular mitochondrial DNA without DSBs, is an indication of mitochondrial damage or mitochondrial affectation.

8. *In vitro* method, according to claim 7, wherein the mitochondrial damage or mitochondrial affectation is a consequence of mitochondrial DNA mutations or depletion, or due to dysfunction of nuclear genes involved in mitochondria structure and physiology, or associated to diseases like diabetes, Huntington's disease, cancer, Alzheimer's disease, Parkinson's disease, bipolar disorder, schizophrenia, aging and senescence, anxiety disorders, cardiovascular disease, sarcopenia, chronic fatigue syndrome, or due to the effect of drugs, physical, chemical or biological agents, or to other conditions that may affect health.

9. Aliquot preparation kit comprising:
a) Aliquot comprising the circular DNA;
b) Aliquot comprising the circular DNA to be analysed along with a first endonuclease enzyme which cleaves one of the two DNA segments of the circular DNA molecule which is located between the at least two specific target sequences to be detected, and
c) Aliquot comprising the circular DNA to be analysed along with a second endonuclease enzyme which cleaves the other DNA segment of the circular DNA molecule which located between the at least two specific target sequences to be detected.

10. Digital PCR kit comprising the aliquot preparation kit of claim 9.

11. Use of the digital PCR kit according to claim 10 for assessing the integrity of a circular DNA molecule.

12. Use, according to claim 11, wherein the integrity of the circular DNA molecule is assessed by determining the presence of at least one DSB in a circular DNA molecule.

13. Use, according to any of the claims 11 or 12, wherein the circular DNA molecule is a mitochondrial DNA molecule.
